# EUROPÄISCHE PATENTSCHRIFT

(11) **EP 1 738 166 B1**
(45) Veröffentlichungstag und Bekanntmachung des Hinweises auf die Patenterteilung: **08.09.2010**
(21) Anmeldenummer: 05742267.7
(22) Anmeldetag: 21.04.2005
(51) Int. Cl.: G01N 33/00, G01N 27/414

(54) **VERFAHREN UND EINRICHTUNG ZUR ERHÖHUNG DER SELEKTIVITÄT VON FET-BASIERTEN GASSENSOREN**
METHOD AND DEVICE FOR INCREASING THE SELECTIVITY OF FET-BASED GAS SENSORS
PROCEDE ET DISPOSITIF POUR AUGMENTER LA SELECTIVITE DE CAPTEURS DE GAZ A BASE DE TRANSISTORS A EFFET DE CHAMP

(30) Priorität: 22.04.2004 DE 102004019640
(43) Veröffentlichungstag der Anmeldung: 03.01.2007
(73) Patentinhaber: Micronas GmbH, 79108 Freiburg i. Br. (DE)
(72) Erfinder: FLEISCHER, Maximilian, 85635 Höhenkirchen (DE); LAMPE, Uwe, 21614 Buxtehude (DE); MEIXNER, Hans, 85540 Haar (DE); POHLE, Roland, 85570 Herdweg (DE); SCHNEIDER, Ralf, 80639 München (DE); SIMON, Elfriede, 80639 München (DE)
(74) Vertreter: Koch Müller Patentanwaltsgesellschaft mbH
(86) Internationale Anmeldenummer: PCT/EP2005/004281
(87) Internationale Veröffentlichungsnummer: WO 2005/103680

(56) Entgegenhaltungen:
- EP-A- 1 059 528
- EP-A- 1 104 884
- DE-C1- 19 517 144
- US-A- 4 633 704
- US-A1- 2002 092 974
- US-B1- 6 454 834
- SUBRENAT A ET AL: "Electrical behaviour of activated carbon cloth heated by the joule effect: desorption application" CARBON, Bd. 39, Nr. 5, April 2001 (2001-04), Seiten 707-716, XP004319893 ISSN: 0008-6223

## Beschreibung

Die Erfindung betrifft ein Verfahren und eine Einrichtung zur Erhöhung der Selektivität von Gassensoren, die mittels Feldeffekttransistor ausgelesen werden. Damit wird die Auslesung schwacher Signale ermöglicht, die beispielsweise von gassensitiven Schichten generiert werden, ohne dass andere eventuell stärkere Gassignale diese stören. Derartige Sensoren weisen Betriebstemperaturen zwischen Raumtemperatur und etwa 100°C auf.

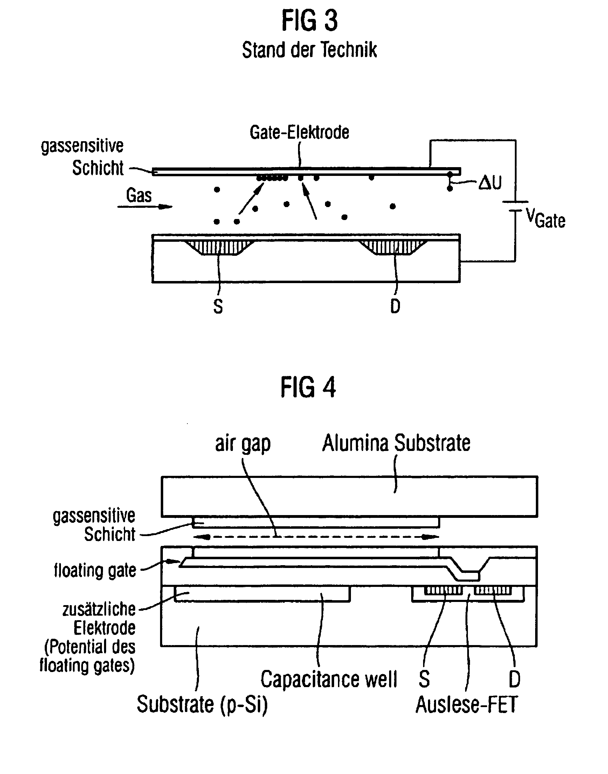

Neuartige FET-Gassensoren in hybridem Aufbau, beispielsweise nach [I-III] oder nach Figur 3 oder 4, welche mittels eines Feldeffekttransistors die Austrittsarbeit eines gassensitiven Materials auslesen, besitzen eine ganze Reihe von Eigenschaften, die ihnen ein überragendes Anwendungspotential zukommen lassen können. Zum einen können sie auch bei Umgebungstemperatur oder bei wenig erhöhten Temperaturen betrieben werden und erlauben daher den Low-Power/Geringe-Betriebsleistung Betrieb mit Batterien oder den direkten Anschluss an Datenbusleitungen ohne die Verwendung von Hilfsenergien. Zum anderen kann bei diesem Sensoraufbau eine große Anzahl von verschiedenen Materialien als Detektionsmaterial eingesetzt werden, was zur Folge hat, dass mit diesen Sensoren eine bisher unerreichte Bandbreite von Gasen detektiert werden kann. Aufgrund des einfachen Aufbaus mit gut automatisierbarer Aufbautechnik ist eine kostengünstige Herstellung möglich. Da die Ansteuerelektronik weitgehend kostenneutral in den Si-Chip integriert werden kann, sind die Kosten für damit aufgebaute Gassensorsysteme geringer als bei anderen Sensortechnologien.

Allerdings unterliegen auch diese Gassensoren der Problematik der Querempfindlichkeiten, d.h. andere in der Anwendung existierende Gase können Störungen des Sensorsignals bewirken. Dabei können zum einen sogenannte direkte Querempfindlichkeit auftreten, d.h. der Sensor reagiert auch auf Störgase, was damit eine Konzentration des Messgases vortäuschen kann. Zum anderen ist mit den sogenannten indirekten Querempfindlichkeiten zu rechnen, d.h. die Sensitivität auf das Zielgas kann durch die Präsenz eines Störgases verändert werden. Beide Effekte führen zu einer Verfälschung des Anzeigewertes und können je nach Anforderungsprofil die Anwendbarkeit in einer Applikation beeinträchtigen oder gar verhindern.

Erster Ansatz zur Beseitigung bestehender Nachteile ist ein oftmals versuchter Lösungsansatz mit einer intelligenten Signalverarbeitung auf Systemebene. Hier wird versucht durch beispielsweise eine Plausibilitätsbetrachtung des Sensorsignals die Folgen von Fehlmessungen zu beheben. Das ist natürlich nicht bei der indirekten Querempfindlichkeit möglich.

Der zweite Ansatz ist anders gestaltet und betreibt die Verwendung eines zusätzlichen Sensorelementes, welches auf das Zielgas sensitiv ist und beim Versuch mit dieser Hilfsinformation den Anzeigewert des Sensorelementes korrigiert. Dies ist ein Ansatz der gerade bei arrayfähigen Sensoren (Mehrfachanordnung) wie den GasFETs (Gassensoren auf Feldeffekttransistoren-Basis), siehe [III], verfolgt wurde. Diese Variante ist immer mit deutlich erhöhtem Aufwand und Kosten verbunden. Die Folgen der indirekten Querempfindlichkeit lassen sich jedoch kaum oder nur mit hohem Aufwand, beispielsweise mit großen Sensorarrays oder umfangreichen Kalibrierungsmodellen, beseitigen.

Der dritte Ansatz besteht darin, das Sensormaterial so weiter zu entwickeln und zu optimieren, dass mit ihm eine selektive Detektion des Zielgases erreicht wird. Dies kann für einige spezielle Applikationen erreicht werden, [IV-VI]. Es kann jedoch nicht davon ausgegangen werden, dass dies für die Mehrzahl von Detektionsaufgaben möglich ist.

Der vierte Ansatz des Standes des Technik bezieht sich direkt auf die FET-Gasensoren, [V]. Er nutzt die Geometrie dieses Aufbaus, um mittels des Anlegens von handhabbaren Spannungen von beispielsweise 10V an dem suspended Gate (abgehobene Gate-Elektrode) aufgrund des kleinen Luftspaltes sehr hohe elektrische Feldstärken an der Oberfläche der Sensorschicht zu erzeugen. Diese beeinflussen das Adsorptionsverhalten der detektierten Moleküle. Aus dem Vergleich des Messsignals bei verschiedenen elektrischen.Feldstärken an der Oberfläche der Sensorschicht lässt sich der Einfluss eines Störgases eliminieren. Diese zeigt schon, dass es sich hier sicherlich um kein universell anwendbares Verfahren handelt, so dass dessen Anwendbarkeit auf wenige Spezialfälle beschränkt bleiben wird.

Der fünfte Ansatz beinhaltet ein selbsterklärendes Vorgehen um Querempfindlichkeiten zu beseitigen. Hierzu wird die Verwendung von Filtern vorgeschlagen, die zwischen dem zu detektierenden Gasgemisch und dem Gassensor angebracht sind. Sie sind permeabel für das Zielgas, lassen aber Gase welche Querempfindlichkeiten verursachen nicht zum Sensor gelangen. Ausführungsformen sind hier katalytische Filter, [IV], wobei die Störgaskonzentrationen aktiv durch eine chemische Reaktion entfernt wird. Oftmals werden Gassensoren auf der Basis geheizter, halbleitender Metalloxide mit einem Aktivkohlefilter kombiniert. Dieser entfernt Gase, welche Querempfindlichkeiten verursachen, indem diese an der großen inneren Oberfläche des Materials adsorbiert werden, wobei das Zielgas aber durchgelassen und vom Sensor detektiert wird. Ein verbreitetes Beispiel für derartige Sensoren sind Gassensoren zur Detektion toxischer, beispielsweise CO, oder explosiver Gase, beispielsweise austretendes Erdgas/CH₄, in Haushaltsatmosphären. Deren Messsignal wird oftmals durch im Haushalt auftretende Alkoholdämpfe gestört , [IV]. Auch für elektrochemische Gassensoren werden Filter häufig eingesetzt. In diesen Anwendungsfällen hat Aktivkohle einen sehr guten Adsorber, z.B. für Alkoholdämpfe, im Langzeitbetrieb. Dabei kann es jedoch zu einer Sättigung des Filters kommen, so dass der Filter seine Wirkung verliert und die Störgase schließlich doch zum Sensor kommen und detektiert werden.

Der Artikel "Electrical behaviour of activated carbon cloth heated by the joule effect: A Desorption Application" by A. Subrenat et al. (Carbon 39 (2001) 707-716) beschreibt die in-situ-Regeneration von Aktivkohlefiltern mittels Erwärmung unter Verwendung des Joule-Effektes.

Der EP 1 059 528 A2 ist ein Gassensor nach dem Prinzip der Austrittsarbeitsmessung zu entnehmen, welcher insbesondere vom zu detektierenden Gas durch eine Filter- oder Schutzschicht getrennt sein kann, welche schädliche Gaskomponenten abhält.

Aus der US 6,454,834 ist ein regenerierbarer Luftreiniger, basierend auf einem Aktivkohlefilter, der durch Erhitzen regeneriert wird, bekannt.

Aus der US 4,633,704 ist es bekannt, vor einem Gassensor einen Filter anzuordnen, der seinerseits hinter einer Diffusionsbarriere liegt, um durch die entsprechende Flussreduktion die Lebensdauer des Filters zu erhöhen.

Weitere Verfahren, mit denen eine Diffusionsbegrenzung zum Schutz eines Filters für eine Messzelle erreicht werden kann, lassen sich der DE 195 17 144 entnehmen.

Aus der EP 1 104 884 ist ein Feldeffekttransistor zur Gasdetektion bekannt, dem ein oder mehrere Gasfilter vorgeschaltet sind, um Täuschungen verursachende Gaskomponenten herauszufiltern, wobei es sich konkret bspw. um Aktivkohlefilter handeln kann.

Der Erfindung liegt die Aufgabe zugrunde bei regenerierbaren FET-basierten Gassensoren eine Verfälschung des Messsignals durch Querempfindlichkeiten möglichst dauerhaft zu vermeiden und den Regenerationsprozess zu überwachen.

Die Lösung dieser Aufgabe geschieht verfahrensmäßig durch die Merkmale gemäß Anspruch 1 und gegenständlich durch die Merkmale des Anspruchs 14. Vorteilhafte Ausgestaltungen sind den Unteransprüchen zu entnehmen.

Die Erfindung geht von einem Vorgehen gemäß dem fünften Ansatz nach dem Stand der Technik aus, wobei ein Filter vor dem Gassensor vorhanden ist, gibt jedoch in Zusammenhang mit speziellen Eigenschaften eines FETs Möglichkeiten zur Umgehung des Problems der Deaktivierung von Filtermaterialien an.

Der Erfindung liegt die Erkenntnis zugrunde, dass FET basierte Gassensoren, die im Gegensatz zum Stand der Technik eine sehr niedrige Betriebstemperatur, beispielsweise Raumtemperatur, aufweisen und mittels vorgeschalteter Filter von Querempfindlichkeiten aufgrund von Störgasen befreit sind, bezüglich der Lebensdauer eines Sensors wesentlich stabilisierbar sind. Dies geschieht durch den Einsatz von bspw. gewebeartigen Aktivkohlefiltern, die mittels mäßiger Temperaturerhöhung regenerierbar sind. Somit kann die Filterlebensdauer wesentlich erhöht werden.

Zusätzlich wird erfindungsgemäß das Messsignal während des Regenerationsprozesses zu dessen Überwachung verwendet. Nachdem die aus dem Filter austretenden Störgase üblicherweise eine signifikante Sensorreaktion, auch bei den erhöhten Betriebstemperaturen, bewirken, wird ein Sensorausschlag vorliegen, der mit dem Fortschritt des Regenerationsprozesses zurückgehen wird. Damit kann überwacht werden, wann der Regenerationsprozess abgeschlossen ist.

### Vorteile

Elimination von Querempfindlichkeiten zur Steigerung der Detektionssicherheit der Gassensoren.

Lösung des Problems mangelnder Langzeitstabilität.

Kostengünstiger Aufbau ohne aufwendige zusätzliche Funktionselemente.

Im Folgenden werden anhand von begleitenden, die Erfindung nicht einschränkenden Figuren Ausführungsbeispiele beschrieben.
- Figur 1: zeigt einen GasFET mit einem erfindungsgemäß nicht beabstandet angebrachten flexiblen Aktivkohlefil- ter.
- Figur 2: zeigt eine schematische Darstellung des Aufbaus zur Verlangsamung der Filtersättigung durch Begrenzung des Gaszutritts, die nicht Teil der Erfindung ist,
- Figur 3: zeigt einen schematischen Grundaufbau eines GasFETS nach dem Stand der Technik (SGFET, suspended Gate FET),
- Figur 4: zeigt einen schematischen Grundaufbau eines GasFETS nach dem Stand der Technik (CCFET, capacitative coupled FET)
- Figur 5: zeigt eine vorteilhafte Ausführung des Verfahrens nach Fig. 2, welches nicht Teil der Erfindung ist, bei der der Gasfilter in eine Vertie- fung der Keramik eingebracht ist.

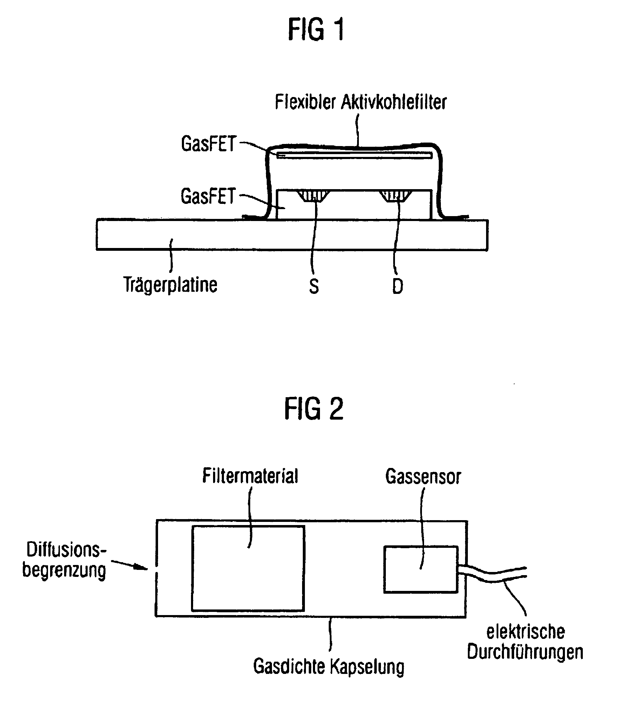

In der Fig. 1 ist im Schnitt ein FET dargestellt, der aus einem Si-Basiskörper mit Source, Drain und Kanal-Bereich mit abgehobener Gate-Elektrode besteht. Dieser GasFET ist mit einem erfindungsgemäß nicht beabstandet angebrachten flexiblen Aktivkohlefilter versehen, der die gesamte Sensoranordnung umschließt.

Fig. 2 zeigt die schematische Darstellung des Aufbaus zur Verlangsamung der Filtersättigung durch Begrenzung des Gaszutritts, die nicht Teil der Erfindung ist, so dass der Filter eine wesentlich längere Betriebszeit aufweist.

In Fig. 3. Wird der Stand der Technik dargestellt in Form des schematischen Grundaufbaus eines GasFET, Typ SGFET

In Fig. 4. Wird der Stand der Technik dargestellt in Form des schematischen Grundaufbaus eines GasFET, Typ CCFET

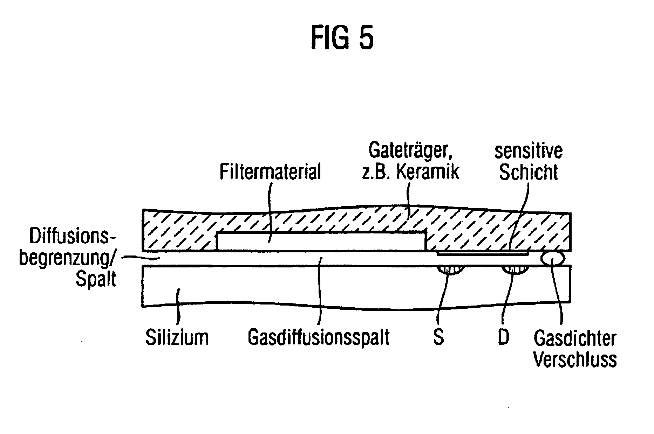

Fig. 5. stellt eine vorteilhafte Ausführung des Verfahrens entsprechend Fig. 2 dar, die nicht Teil der Erfindung ist, bei der der Gasfilter in eine Vertiefung der Keramik eingebracht ist. Die Diffusionsbegrenzung geschieht durch die entsprechend kleine Auslegung des Diffusionsspaltes.

### Filterregenerationsverfahren

Der erfindungsgemäße lösungsweg verwendet die Eigenschaft von GasFETs, dass deren Betriebstemperaturen drastisch unter denen der geheizten Metalloxidsensoren liegen und sich typischerweise von Raumtemperatur bis hin zu etwa 100°C erstrecken.

Bevorzugt wird hier ein Filter verwendet, der ohne räumlichen oder nur mit geringem räumlichen Abstand zum eigentlichen Sensorelement angebracht ist. Ein typischer Aufbau ist in Fig. 1 gezeigt. Nachdem die Betriebstemperatur des GasFET wie eben erwähnt maximal ca. 100°C beträgt, kann der Filter die Betriebstemperatur des GasFET annehmen, ohne dass seine adsorbierende Wirkung unzulässigerweise abnimmt.

Ein geeignetes Filtermaterial sind Gewebe aus Kohlefasern, wie sie z.B. von Charcoal Cloth unter dem Markennamen Zorflex™ vertrieben werden. Hergestellt werden die Gewebe aus reinem Viskosezellstoff, der unter entsprechenden Reaktionsbedingungen vollständig verkohlt wird. Bedingt durch den Aufbau zeichnet sich das Material durch eine große aktive Oberfläche aus, wobei die aktive Oberfläche im wesentlichen durch die Mikroporen innerhalb der Fasern hergestellt wird. Weitere Eigenschaften sind eine hohe mechanische Flexibilität und Stabilität, ein geringes Gewicht und eine große chemische Resistenz. Da die Filter vor der Verkohlung als Gewebe hergestellt werden, sind die Eigenschaften über einen weiten Bereich variierbar, beispielsweise über die Faserlänge und - dicke, die Struktur des Gewebes, Gewebestärke usw.. Die Gasadsorption wird als reine Physisorption beschrieben. Dadurch kann mit einer Erhöhung der Temperatur um z. B. 100 °C das Material durch Desorption aller Adsorbate wieder regenerieren. Spezifische Imprägnierungen erhöhen die Filterwirkung für bestimmte saure oder basische Gase deutlich. Diese Gase werden dann durch Chemisorption gebunden. Derartige Gewebe werden mit Erfolg eingesetzt, z. B als Schutzkleidung, zur Wasserreinigung, als Ölfilter zur Reinigung von Pressluft, zum Schutz von Kunstwerken vor korrosiven Gasen, als Filter in Gasmasken.

Die GasFET-Sensoren sind üblicherweise mit einem Heizelement versehen, um die Temperatur zu stabilisieren. Bevorzugt wird nun dieses Heizelement benutzt, um den Aufbau, den Gassensor mit dem direkt darauf aufgebrachten Filter, auf eine gegenüber der Betriebstemperatur erhöhte Temperatur zu heizen. Typische Temperaturen liegen hier im Bereich von 200-300°C. Dadurch, dass Filter und Sensor in direkten Kontakt miteinander sind, erfolgt ein starker Wärmeübergang vom GasFET auf den Filter und dieser erreicht die Temperatur des Gassensors. Bei diesen Temperaturen findet durch die thermische Aktivierung ein Ausgasen des Aktivkohlefilters statt. Die adsorbierten Gase verlassen den Filter, wodurch dessen volle Adsorptionskapazität wieder erreicht wird, d.h. es findet eine Filterregeneration statt. Typische Perioden für die Durchführung des Regenerationsprozesses liegen bei 1-20 Tagen. Typische Zeitdauern für diesen Regenerationsprozess liegen bei 0,5 bis 3 min. Während des Regenerationsprozesses darf vom Gassensor kein aussagefähiges Sensorsignal erwartet werden, da sowohl der GasFET bei einer zu hohen Temperatur betrieben wird, als auch durch die erzwungene Desorption eine gewisse Menge der Störgase auch zum GasFET gelangen kann.

Zusätzlich wird erfindungsgemäß das Messsignal wahrend des Regenerationsprozesses zu dessen Überwachung verwendet. Nachdem die aus dem Filter austretenden Störgase üblicherweise eine signifikante Sensorreaktion, auch bei den erhöhten Betriebstemperaturen, bewirken, wird ein Sensorausschlag vorliegen, der mit dem Fortschritt des Regenerationsprozesses zurückgehen wird. Damit kann überwacht werden, wann der Regenerationsprozess abgeschlossen ist.

Eine Variante, die nicht Teil der Erfindung ist, basiert sowohl auf konstruktiven Details der FET-Gassensoren wie auch auf ihrer Eigenschaft, im Gegensatz zu den stark Gas konsumierenden Metalloxidsensoren, nur eine sehr geringe Menge an Analysegas zur Detektion zu benötigen. Um jetzt die Lebensdauer des Filterelementes zu erhöhen bzw. die Deaktivierung durch Sättigung zu vermeiden, wird der Gaszutritt zum Filterelement stark begrenzt. Dadurch wird die Filtersättigung deutlich hinausgezögert. Die kleine Menge an verfügbaren Gas reicht jedoch zur Gasdetektion mit dem FET-Sensor aus. Eine schematische Beschreibung ist in Fig. 2 gezeigt.

Die Begrenzung der Gasdiffusion kann sowohl durch ein kleines Loch als Diffusionsbegrenzung als auch durch eine diffusionsbegrenzende Membran erreicht werden, welche zudem noch das Systems gut gegen Staub schützt. Eine vorteilhafte Ausführung dieses Prinzips gibt die Fig. 5. an. Gezeigt ist hier der Bereich des Gasdiffusionsspaltes in einem hybriden Flip-Chip Aufbau für einen Gassensor. Es wird eine Vertiefung in das Trägermaterial des gassensitiven Gates eingebracht, in der dann das Filtermaterial deponiert wird. Dadurch wird der Diffusionsspalt verlängert. Die Begrenzung der Gasdiffusion geschieht nun durch den ersten Bereich des Gasdiffusionsspalts. Aus der verminderten Gasmenge werden vom Filtermaterial alle störende Gaskomponenten nahezu vollständig entfernt und das gereinigte Gas gelangt nun zur Detektionsschicht.

### Für beide Varianten gilt gleichermaßen:

- Die Vorgehensweisen werden typischerweise für Querempfindlichkeiten gegenüber NOx, NH₃, Alkoholen oder Ketonen angewandt, welche wesentliche Störgase für Festkörpergassensoren darstellen. Für diese Gase sind sehr effektive Adsorptionsfilter erhältlich.
- Falls erforderlich können auch mehrere Lagen Aktivkohlefilter verwendet werden. Es gibt z.B. mit saueren Materialien imprägnierte Aktivkohlefilter, welche gut das basische NH₃ adsorbieren, und mit alkalischen Materialien imprägnierte Filter, welche sehr gut das sauere NO₂ binden.
- Das Verfahren kann unter Verwendung Feuchte adsorbierender Filterschichten wie beispielsweise Silika-Gel auch verwendet werden, um Feuchteschwankungen auszugleichen. Ziel ist hier nicht die vollständige Entfernung der Feuchte, sondern die Verwendung der Filterschicht als Puffer, welcher auch Feuchte abgeben kann, um die die Gasdetektion störenden Feuchteschwankungen zu glätten.

### Literatur

[I] T. Doll, B. Flietner, I Eisele, DE 4239319,
[II] M. Fleischer, U. Lampe, B. Ostrick, H. Meixner, F. Daeche, DE 19956744,
[III] M. Fleischer, B. Ostrick, H. Meixner, DE 19956806,
[IV] B. Ostrick, M. Fleischer, H. Meixner, DE 19926747, E. Simon, M. Fleischer, H. Meixner; Porphin-dyes - high potential N02-layers for asthma detection in breath in workfunction type gas sensors. Proceedings Eurosensors XVI, Prague, 15.-18.9.2002, pp. 647
[V] B. Ostrick, M. Bögner, M. Fleischer, H. Meixner, T. Doll. I. Eisele, DE19849932.
[VI] G. Flingelli, M. Fleischer, H. Meixner, DE 19708770; Produktschrift der Figaro Engineering, Figaro Gassensor TGS203

## Patentansprüche

1. Verfahren zur Erhöhung der Selektivität eines FET basierten Gassensors, bei dem zwischen dem zu detektierenden Gasgemisch und dem Gassensor mindestens ein zielgasdurchlässiger Filter mit großer aktiver Oberfläche vorhanden ist, der durch pulver- oder gewebeförmige Aktivkohle dargestellt wird, **dadurch gekennzeichnet, dass** der Filter zur Filterregeneration zeitweise auf eine Temperatur aufgeheizt wird, bei der adsorbierte Gase ausgetrieben werden, und dass das Sensorsignal während der Filterregeneration zur Steuerung der Regenerationszeit verwendet wird.

2. Verfahren nach Anspruch 1, bei dem die Betriebstemperatur des Gassensors im Bereich zwischen Raumtemperatur und etwa 100°C liegt.

3. Verfahren nach einem der Ansprüche 1 oder 2, bei dem Filter mit geringem oder keinen räumlichen Abstand zum Sensorelement platziert sind.

4. Verfahren nach einem der Ansprüche 1 bis 3, bei dem das gewebeförmige Aktivkohlematerial aus Viskosezellstoff durch Verkohlung hergestellt ist.

5. Verfahren nach einem der Ansprüche 1 bis 4, bei dem die Filterwirkung durch Physisorption erreicht wird.

6. Verfahren nach einem der Ansprüche 1 bis 5, bei dem die Aufheizung von Filtern zusammen mit der Sensorheizung geschieht.

7. Verfahren nach einem der Ansprüche 1 bis 6, bei dem eine Filterregeneration bei einer Temperatur von 100°C über der Betriebstemperatur des Sensors durchgeführt wird.

8. Verfahren nach einem der Ansprüche 1 bis 6, bei dem eine Filterregeneration in einem Temperaturbereich von etwa 150 - 300°C durchgeführt wird.

9. Verfahren nach einem der Ansprüche 1 bis 8, bei dem Filter-Regenerationszyklen automatisch gesteuert werden.

10. Verfahren nach einem der vorhergehenden Ansprüche, bei dem mehrere Lagen von Filtern verwendet werden.

11. Verfahren nach einem der vorhergehenden Ansprüche, bei dem die Filter imprägniert sind.

12. Verfahren nach einem der vorhergehenden Ansprüche, bei dem Filter als Puffer für Feuchtigkeit verwendet werden.

13. Verfahren nach einem der vorhergehenden Ansprüche, bei dem das Sensorsignal während der Filterregeneration zur Realisierung eines Selbsttestes des Sensors verwendet wird.

14. Einrichtung zur Durchführung eines Verfahrens nach einem der Ansprüche 1 bis 13 mit einem FET basierten Gassensor, bei welchem zwischen einem zu detektierenden Gasgemisch und dem Gassensor mindestens ein zielgasdurchlässiger Filter vorhanden ist, wobei der Filter pulver- oder gewebeförmige Aktivkohle aufweist, sowie einer Heizeinrichtung, durch welche der Filter zur Filterregeneration auf eine Temperatur aufheizbar ist, bei der adsorbierte Gase ausgetrieben werden.

## Claims

1. A method of increasing the selectivity of a FET-based gas sensor, in which at least one filter with a large active surface, which is permeable to target gas and which is represented by activated carbon in the form of a powder or fabric, is present between the gas mixture to be detected and the gas sensor, **characterized in that**, for regeneration of the filter, the filter is first heated temporarily to a temperature at which adsorbed gases are expelled, and the sensor signal is used to control the regeneration time during the regeneration of the filter.

2. A method according to claim 1, in which the operating temperature of the gas sensor is in the range between room temperature and approximately 100°C.

3. A method according to one of claims 1 or 2, in which filters are positioned at a short spatial distance or at no spatial distance from the sensor element.

4. A method according to any one of claims 1 to 3, in which the activated-carbon material in the form of a fabric is produced from viscose pulp by carbonization.

5. A method according to any one of claims 1 to 4, in which the filter action is achieved by physisorption.

6. A method according to any one of claims 1 to 5, in which the heating of filters takes place together with the sensor heating.

7. A method according to any one of claims 1 to 6, in which a regeneration of the filters is carried out at a temperature of 100°C above the operating temperature of the sensor.

8. A method according to any one of claims 1 to 6, in which a regeneration of the filters is carried out in a temperature range of from approximately 150 to 300°C.

9. A method according to any one of claims 1 to 8, in which filter-regeneration cycles are controlled automatically.

10. A method according to any one of the preceding claims, in which a plurality of layers of filters are used.

11. A method according to any one of the preceding claims, in which the filters are impregnated.

12. A method according to any one of the preceding claims, in which filters are used as buffers for moisture.

13. A method according to any one of the preceding claims, in which the sensor signal is used to carry out a self test of the sensor during the regeneration of the filters.

14. A device for performing a method according to any one of claims 1 to 13 with a FET-based gas sensor, in which at least one filter permeable to target gas is present between a gas mixture to be detected and the gas sensor, wherein the filter has activated carbon in the form of a powder or fabric, and a heating device by which, for regeneration of the filter, the filter is capable of being heated to a temperature at which adsorbed gases are expelled.

## Revendications

1. Procédé pour augmenter la sélectivité d'un capteur de gaz à base de transistors FET ayant un filtre perméable aux gaz, ciblée, avec une surface à grande activité, entre le mélange de gaz à détecter et le capteur de gaz, ce filtre étant constitué par du charbon actif à l'état de poudre ou de tissu,
procédé **caractérisé en ce que**
pour sa régénération, on chauffe de temps en temps le filtre à une température à laquelle les gaz adsorbés sont expulsés, et
pendant la régénération du filtre, le signal du capteur est utilisé pour commander la durée de la régénération.

2. Procédé selon la revendication 1,
selon lequel
la température de fonctionnement du capteur de gaz se situe dans une plage comprise entre la température ambiante et une température d'environ 100°C.

3. Procédé selon la revendication 1 ou 2,
selon lequel
des filtres sont placés à faible distance de l'élément de capteur ou directement contre celui-ci.

4. Procédé selon l'une des revendications 1 à 3,
selon lequel
la matière constituée par le charbon actif à l'état de tissu, est obtenue par carbonisation d'une matière cellulaire de viscose.

5. Procédé selon l'une des revendications 1 à 4,
selon lequel
l'effet de filtre est obtenu par adsorption physique.

6. Procédé selon l'une des revendications 1 à 5,
selon lequel
on chauffe les filtres ensemble avec l'installation de chauffage.

7. Procédé selon l'une des revendications 1 à 6,
selon lequel
on effectue la régénération des filtres à une température de 100°C supérieure à la température de fonctionnement du capteur.

8. Procédé selon l'une des revendications 1 à 6,
selon lequel
on effectue la régénération du filtre dans une plage de températures de l'ordre de 150-300°C.

9. Procédé selon l'une des revendications 1 à 8,
selon lequel
les cycles de régénération des filtres sont commandés automatiquement.

10. Procédé selon l'une des revendications précédentes,
selon lequel
on utilise plusieurs couches de filtres.

11. Procédé selon l'une des revendications précédentes,
selon lequel
on imprègne les filtres.

12. Procédé selon l'une des revendications précédentes,
selon lequel
on utilise des filtres comme tampons pour l'humidité.

13. Procédé selon l'une des revendications précédentes,
selon lequel
pendant la régénération des filtres, on utilise le signal de capteur pour réaliser un autocontrôle du capteur.

14. Installation pour la mise en oeuvre d'un procédé selon l'une des revendications 1 à 13 pour un capteur de gaz à base de transistors FET, ayant au moins un filtre perméable aux gaz de manière ciblée, entre un mélange de gaz à détecter et le capteur de gaz,
le filtre comportant du charbon actif à l'état de poudre ou de tissu, ainsi qu'une installation de chauffage pour chauffer les filtres pour leur régénération, à une température à laquelle les gaz adsorbés sont expulsés.
